# EUROPÄISCHE PATENTSCHRIFT

(11) **EP 0 515 769 B1**
(45) Veröffentlichungstag und Bekanntmachung des Hinweises auf die Patenterteilung: **23.08.1995**
(21) Anmeldenummer: 92100120.2
(22) Anmeldetag: 07.01.1992
(51) Int. Cl.: A61F 5/14

(54) **Orthopädische Schuheinlage zur Korrektur der Grosszehen-Adduktion**
Orthopedic insole for correcting adduction of the big toe
Semelle orthopédique pour correction de l'adduction du gros orteil

(30) Priorität: 31.05.1991 DE 9106735 U
(43) Veröffentlichungstag der Anmeldung: 02.12.1992
(73) Patentinhaber: SOLOR SCHUHFORSCHUNG UND ENTWICKLUNG GmbH, D-66953 Pirmasens (DE)
(72) Erfinder: Birke, Josef, W-6780 Pirmasens/Pfalz (DE)
(74) Vertreter: Patentanwälte Möll und Bitterich

(56) Entgegenhaltungen:
- FR-A- 459 824
- FR-A- 945 677
- FR-A- 1 045 725
- US-A- 1 665 030

## Beschreibung

Die Erfindung betrifft eine individuell anpaßbare orthopädische Schuheinlage aus Kunststoff zur Korrektur der Adduktion der Großzehe gemäß dem Oberbegriff des Anspruchs 1.

Eine derartige Schuheinlage ist aus der WO-A-9 107 152 bekannt.

Eine meist angeborene Abnormität der Füße ist eine Fehlstellung der Großzehe, die "affenartig" absteht. In den meisten Fällen ist es möglich, die Adduktion der Großzehe durch spezielle Korrektureinlagen, die in orthopädischen Schuhen getragen werden, endgultig zu korrigieren. Diese Einlagen werden auf Verschreibung des Arztes nach Maß gefertigt. Sie bestehen üblicherweise aus einer fußbettartigen Rückfußschale, an der seitlich neben der Großzehe ein Korrekturflügel angeformt ist. Dieser wird so gerichtet, daß die Großzehe in die gewünschte Richtung parallel zu den übrigen Zehen gedrückt wird. Mit fortschreitendem Korrekturerfolg werden immer wieder neue Einlagen angefertigt, da Vorrichtungen zum Nachstellen insbesondere des Korrekturflügels bei dieser Art von Einlagen nicht anbringbar sind. Dies ist mit erheblichen Kosten verbunden.

Ein weiterer Nachteil der bekannten Korrektureinlagen besteht darin, daß der nach vorne gerichtete Korrekturflügel aufgrund der Abrollbewegung beim Gehen leicht abbricht bzw. die Ferse im Schuh hochhebelt.

Außer den hier relevanten Schuheinlagen sind für schwere Fälle auch Korrekturschienen bekannt, die am Bein und Fuß befestigt werden. Diese sind meist mit geeigneten Nachstelleinrichtungen ausgerüstet. Allerdings können diese Korrekturschienen nicht in Schuhen getragen werden.

Der vorliegenden Erfindung liegt die Aufgabe zugrunde, eine Schuheinlage für die Adduktionskorrektur der Großzehe der eingangs genannten Art anzugeben, die einen verbesserten, insbesondere schnelleren Heilerfolg verspricht, ohne großen Aufwand an den Fuß des Patienten anpaßbar ist und dem Korrekturerfolg entsprechend nachgestellt werden kann.

Diese Aufgabe wird gelöst durch eine gattungsgemäße Schuheinlage mit den Merkmalen gemäß Kennzeichen des Anspruchs 1.

Dank der Herstellung aus thermoplastischem Kunststoff ist zunächst eine Serienfertigung von normierten Rohlingen der Korrektureinlage im Spritzgußverfahren möglich. Die Anpassung an die individuelle Fußform des Patienten erfolgt in einfachster Weise durch Auflegen des erwärmten Rohlings auf einen Leisten oder Gipsabdruck des Patienten. Bei dieser Vorgehensweise ist eine Nachbearbeitung, beispielsweise durch Materialabnahme, entbehrlich.

Dank der Herstellung aus elastischem Kunststoff wird die Abrollbewegung von Fuß und Schuh beim Gehen nicht behindert, was noch dadurch unterstützt wird, daß die Unterkante des Korrekturflügels konvex gekrümmt ist.

Der wesentliche Vorteil der vorliegenden Erfindung liegt jedoch darin, daß durch die Kombinationswirkung aller Merkmale der Korrekturflügel aufgrund der Abrollbewegung beim Gehen jedesmal um einen bestimmten Winkel nach innen geschwenkt wird. Die Adduktion der Großzehe wird also aktiv korrigiert, wodurch nach allen medizinischen Erkenntnissen eine schnellere Ausheilung möglich ist.

Gemäß einer vorteilhaften Weiterbildung der Erfindung ist im übergangsbereich zwischen Rückfußschale und Korrekturflügel, d. h. etwa im Ballenbereich, wenigstens eine Stabilisierungsrippe angeformt. Diese verhindert im Zusammenhang mit dem Schuh, daß der Korrekturflügel durch die Kombinationswirkung von Großzehendruck und Abrollbewegung nach außen wegschwenkt.

Unterstützt wird die aktive Korrekturbewegung, d. h. das Einwärtsschwenken des Korrekturflügels, dadurch, daß gemäß einer vorteilhaften Weiterbildung der Erfindung die Vorderkante der Rückfußschale, vom Korrekturflügel ausgehend, leicht nach vorne gezogen ist, so daß der Außenstrahl der Rückfußschale länger ist als der Ballenauftritt. Als optimal hat sich ein Winkel bis ca. 10 Grad herausgestellt. Beim Abrollen des Fußes kippt die Rückfußschale und mit ihr der Fuß des Patienten um diese leicht schräg gestellte Vorderkante, wodurch der Korrekturflügel und mit ihm die zu korrigierende Großzehe um einen entsprechenden Betrag zusätzlich einfedert.

Gemäß einer Ausgestaltung der Erfindung ist der Korrekturflügel um einen Winkel von etwa 5 bis 15 Grad gegen die Senkrechte nach außen geneigt. Bei einer stärkeren Neigung besteht die Gefahr, daß die Großzehe trotz der konkaven, schalenartigen Ausformung der Innenfläche des Korrekturflügels den Kontakt zum Korrekturflügel verliert.

Eine optimale Materialauswahl ist dann gegeben, wenn der Kunststoff zusätzlich bruchfest ist.

Anhand der Zeichnung soll die Erfindung in Form eines Ausführungsbeispiels näher erläutert werden. Es zeigen
- Fig. 1: eine schematische, perspektivische Ansicht einer Korrektureinlage von schräg vorne oben und
- Fig. 2: die entsprechende Darstellung der Korrektureinlage nach Fig. 1 von schräg hinten oben.

Die Figuren zeigen eine orthopädische Schuheinlage zur Korrektur der Adduktion der Großzehe. Sie besteht aus einem thermoplastischen, elastischen und vorzugsweise bruchfesten Kunststoff und kann infolgedessen als Serienfertigung im Spritzgießverfahren hergestellt werden, wobei die Materialverteilung entsprechend den Festigkeitsanforderungen optimal gewählt werden kann.

Die Schuheinlage besteht aus einer fußbettartigen Rückfußschale 1, deren Vorderkante 2 etwa unter dem Großzehenballen liegt. Seitlich angeformt und unter einem Winkel von bis 15 Grad gegen die Senkrechte nach außen geneigt erkennt man einen Korrekturflügel 3. Dessen die Großzehe seitlich außen stützende Innenfläche 7 ist leicht konkav, d. h. schalenartig gewölbt, so daß die Großzehe gestützt und geführt wird, ohne fest fixiert zu sein.

Im Übergangsbereich zwischen Rückfußschale 1 und Korrekturflügel 3, d. h. etwa im Bereich des Großzehenballens, sind außen drei Versteifungsrippen 4 angeformt. Diese sollen verhindern, daß der Korrekturflügel 3 nach außen weggeschwenkt wird, beispielsweise durch den Druck der Großzehe sowie die noch zu beschreibenden dynamischen Vorgänge beim Gehen.

Die Unterkante 5 des Korrekturflügels ist konvex nach oben gekrümmt. So kann der Korrekturflügel 3 die Abrollbewegung beim Gehen nicht behindern.

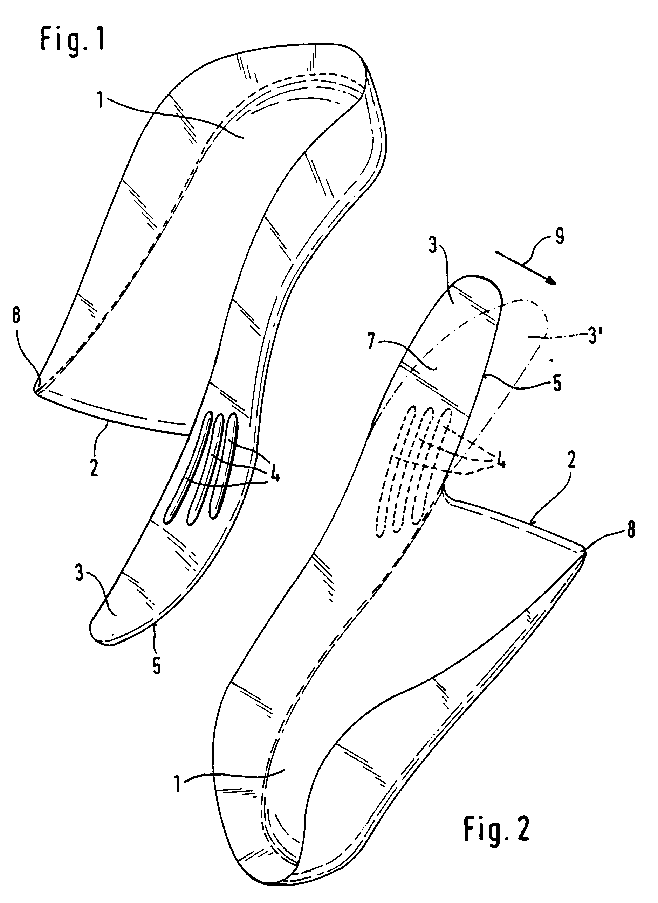

Durch die Kombinationswirkung von Schalenform, Neigung und Unterkantenwölbung des Korrekturflügels 3 wird der Korrekturflügel 3 durch die Abrollbewegung beim Gehen nach innen geschwenkt, wie dies in Fig. 2 durch den Pfeil 9 symbolisiert wird. Der Korrekturflügel 3 erreicht dann die strichpunktierte Position 3' und federt anschließend insbesondere unter dem Druck der Großzehe wieder in seine Ursprungsstellung zurück. Die anliegende Großzehe wird also bei jedem Schritt dynamisch zu der gewünschten Parallelstellung hin gedrückt. Es findet eine aktive Korrektur statt.

Diese aktive Korrekturbewegung des Korrekturflügels 3 läßt sich noch vergrößern, wenn die Vorderkante 2 der Rückfußschale 1 vom Ballenauftritt ausgehend zum Außenstrahl 8 hin leicht, d. h. um einen Winkel bis ca. 10 Grad, nach vorne gezogen wird. Da die im wesentlichen steife Rückfußschale 1 beim Gehen um die entsprechend abgewinkelte Vorderkante 2 kippt, federt der Korrekturflügel 3 um einen entsprechenden Betrag weiter nach innen.

Dank der Herstellung aus thermoplastischem Kunststoff kann der in Großserie im Spritzgießverfahren hergestellte Rohling der Korrektureinlage einfach an die individuelle Fußform der kleinen Patienten angepaßt werden, indem das Kunststoffmaterial erwärmt und anschließend auf den Patientenleisten gepreßt wird. Dank der thermoplastischen Eigenschaften kann der Korrekturflügel mit fortschreitendem Korrekturerfolg durch Erwärmen des Kunststoffmaterials nachgestellt werden.

In jedem Fall erhält man eine preiswerte, leichte und praktisch keinen Platz beanspruchende Korrektureinlage, die in fast allen auch nicht orthopädischen Schuhen getragen werden kann. Es besteht darüber hinaus auch die Möglichkeit, nicht nur eine Adduktionskorrektur der Großzehe durchzuführen, sondern auch die Fehlstellungen der Rückfüße wie Klump-, Platt-, Knick-, Hohlfüße usw. aktiv zu korrigieren.

## Patentansprüche

1. Individuell anpaßbare orthopädische Schuheinlage aus elastischem Kunststoff zur Korrektur der Adduktion der Großzehe, mit einer fußbettartigen Rückfußschale (1), deren Vorderkante (2) etwa unter dem Großzehenballen liegt, und einem seitlich angeformten, etwa senkrecht stehenden, die Großzehe seitlich außen stützenden Korrekturflügel (3), dadurch gekennzeichnet, daß sie aus einem thermoplastischen Kunststoff gespritzt ist, daß der Korrekturflügel (3) leicht nach außen geneigt ist, daß die Innenfläche (7) des Korrekturflügels (3) leicht konkav gewölbt ist und daß die Unterkante (5) des Korrekturflügels (3) konvex gekrümmt ist.

2. Schuheinlage nach Anspruch 1, dadurch gekennzeichnet, daß im Übergangsbereich zwischen Rückfußschale (1) und Korrekturflügel (3) wenigstens eine Stabilisierungsrippe (4) angeformt ist.

3. Schuheinlage nach Anspruch 1 oder 2, dadurch gekennzeichnet, daß die Vorderkante (2) der Rückfußschale (1) - vom Korrekturflügel (3) ausgehend - leicht nach vorn gezogen ist, so daß der Außenstrahl (8) länger ist als der Ballenauftritt.

4. Schuheinlage nach Anspruch 3, dadurch gekennzeichnet, daß die Vorderkante (2) um einen Winkel bis ca. 10 Grad nach vorn gezogen ist.

5. Schuheinlage nach einem der Ansprüche 1 bis 4, dadurch gekennzeichnet, daß der Korrekturflügel (3) etwa 5 bis 15 Grad gegen die Senkrechte nach außen geneigt ist.

6. Schuheinlage nach einem der Ansprüche 1 bis 5, dadurch gekennzeichnet, daß der Kunststoff bruchfest ist.

## Claims

1. An individually adaptable orthopaedic shoe insert of resilient synthetic material for correcting adduction of the big toe, having an orthopaedic-support like tarsal shell (1), the front edge (2) of which is situated approximately under the ball of the big toe, and having a substantially vertically upright corrective flap (3) which is integrally formed laterally and which supports the big toe laterally outwardly, characterised in that it is injection-moulded from a thermoplastic synthetic material, in that the corrective flap (3) is inclined slightly outwards, in that the inner surface (7) of the corrective flap (3) is curved slightly concavely, and in that the lower edge (5) of the corrective flap (3) is convexly curved.

2. A shoe insert according to Claim 1, characterised in that at least one stabilising rib (4) is integrally moulded in the transition zone between the tarsal shell (1) and the corrective flap (3).

3. A shoe insert according to Claim 1 or 2, characterised in that the front edge of the tarsal shell (1) - starting from the corrective flap (3) - is extended slightly forwards so that the outer perimeter (8) is longer than the ball tread.

4. A shoe insert according to Claim 3, characterised in that the front edge (2) is extended forwards through an angle of about 10 degrees.

5. A shoe insert according to any one of Claims 1 to 4, characterised in that the corrective flap (3) is inclined outwards by about 5 to 15 degrees relative to the vertical.

6. A shoe insert according to any one of Claims 1 to 5, characterised in that the synthetic material has high fracture strength.

## Revendications

1. Garniture de chaussure orthopédique susceptible d'être adaptée individuellement et réalisée en une matière synthétique élastique, pour la correction de l'adduction du gros orteil, avec une coque d'arrière-pied (1), servant de base de pied, dont l'arête avant (2) est située à peu près sous le coussinet du gros orteil, et avec une ailette de correction (3), formée d'un seul tenant latéralement, placée à peu près verticalement, soutenant le gros orteil extérieurement et latéralement, caractérisée en ce qu'elle est réalisée par moulage en une matière synthétique thermoplastique, en ce que l'ailette de correction (3) est légèrement inclinée vers l'extérieur, en ce que la face intérieure (7) de l'ailette de correction (3) est légèrement incurvée de façon concave et en ce que l'arête inférieure (5) de l'ailette de correction (3) est incurvée de façon convexe.

2. Garniture de chaussure selon la revendication 1, caractérisée en ce qu'au moins une nervure de stabilisation (4) est formée d'un seul tenant dans la zone de transition entre la coque d'arrière-pied (1) et l'ailette de correction (3).

3. Garniture de chaussure selon la revendication 1 ou 2, caractérisée en ce que l'arête avant (2) de la coque d'arrière-pied (1) est légèrement étirée vers l'avant à partir de l'ailette de correction (3), de sorte que le profil extérieur (8) est plus grand que l'appui du coussinet.

4. Garniture de chaussure selon la revendication 3, caractérisée en ce que l'arête avant (2) est étirée vers l'avant d'un angle allant jusqu'à environ 10 degrés.

5. Garniture de chaussure selon l'une des revendications 1 à 4, caractérisée en ce que l'ailette de correction (3) est inclinée vers l'extérieur d'environ 5 à 15 degrés par rapport à la verticale.

6. Garniture de chaussure selon l'une des revendications 1 à 5, caractérisée en ce que la matière synthétique est résistante à la rupture.
